# EUROPEAN PATENT SPECIFICATION

(11) **EP 0 239 859 B1**
(45) Date of publication and mention of the grant of the patent: **28.01.1998**
(21) Application number: 87103821.2
(22) Date of filing: 17.03.1987
(51) Int. Cl.: C07K 1/02, A61K 35/14, A61K 35/16

(54) **Removal of lipid soluble process chemicals from biological materials by extraction with naturally occurring oils or synthetic substitutes thereof**
Entfernung von fettlöslichen Reagenzien aus biologischen Materialien durch Extrahierung mit natürlichen Ölen oder synthetischen Ersätzen davon
Elimination de réactifs liposolubles dans des matériaux biologiques par extraction avec des huiles naturelles ou des succédanés synthétiques de celles-ci

(30) Priority: 31.03.1986 US 846374
(43) Date of publication of application: 07.10.1987
(73) Proprietor: New York Blood Center, Inc., New York, New York 10021 (US)
(72) Inventor: Woods, Kenneth R., Sea Cliff, NY 11579 (US); Orme, Thomas W., Huntingen Station, NY 11746 (US)
(74) Representative: Cohausz & Florack Patentanwälte

(56) References cited:
- WO-A-83/04371
- DE-A- 3 005 495
- DE-A- 3 310 263
- GB-A- 50 061
- US-A- 3 647 624
- US-A- 4 540 573
- CHEMICAL ABSTRACTS, vol. 96, no. 13, 29 March 1982, Columbus, OH (US); N. SHIBATA et al., p. 516, no. 101990k#
- CHEMICAL ABSTRACTS, vol. 77, no. 11, 11 September 1972, Columbus, OH (US); M.A. EVENSON et al., p. pp. 234-235, no. 72317w#
- Clinical Chemistry Vol. 18(6), 1972, pp. 554-62

## Description

The invention concerns removal of virus attenuating chemicals and other lipid soluble process chemicals from biological materials by extraction with naturally occurring oils or synthetic substitutes thereof.

Numerous attempts have been made to inactivate viruses such as hepatitis B virus (HBV) in mammalian, especially human, blood plasma. Inactivation renders a virus non-infectious and non-pathogenic. It is the practice in some countries to effect inactivation of the hepatitis B virus in the blood plasma by bringing the plasma into contact with a viral inactivating agent which cross links the proteinaceous portions of hepatitis B virus or which interacts with the nucleic acid of the virus. For instance, it is known that hepatitis B virus is inactivated by contact with an aldehyde, such as formaldehyde.

Clinical Chemistry, Vol. 18, No. 6 pages 554-562, 1972 describes a hemodialysis system for the removal of unwanted substances, in particular drugs after a drug intoxication, cholesterol or bilirubin by extraction of blood with oil. In view of the amount of oil to be used at least an equal volume of oil and drug containing solution is proposed.

U.S. 3 647 624 refers to the treatment of blood and other body fluids by liquid/liquid contact with an oleaginous substance. By way of this process lipid soluble drugs and other unwanted substances are extracted from blood and other body fluids. This may be performed by passing droplets of blood through a surplus of oil with flow rates of 8 or 16 ml/min.

U.S. patents 4,481,189 and 4,540,573 describe the use of organic solvent/detergent pairs to reduce by several orders of magnitude the infectivity of hepatitis viruses and certain other viruses contained in plasma and plasma products or added thereto.

Solvent/detergent treatment under appropriate conditions of temperature and contact time effectively disassembles viruses that have envelope proteins associated with lipid, while having negligible effect on the molecular conformation and biological activities of sensitive blood plasma proteins.

The independent effects of organic solvents and detergents in disassembling and attenuating viruses can be facilitated by the presence of both. Removal of detergents, as well as organic solvents, from biologic products may be necessary, especially if a particular detergent is not well tolerated by humans or whatever biologic system within which the product is to be used.

Methods used to achieve removal of lipid/detergent mixed micelles from membrane protein complexes may be applicable to removal of the same from plasma products and other biologic products. These have been based on differences in size. buoyant density, charge, binding affinity, phase partitioning and solvent partitioning (A. Helenius and K. Sinous, "Solubilization of Membrane by Detergents", Biochem. Biophys. ACTA, (1975), 415, 29-79).

The invention refers to a method of removing lipid soluble process chemicals from fluid biological materials selected from the group consisting of blood plasma, Fraction I, Fraction II, Fraction III, Fraction IV-1, Fraction IV-4, Fraction V, Fraction VI, fibronectin, antihemophilic factor, prealbumin, retinol-binding protein, albumin, alpha-globulins, beta-globulins, gamma-globulins, antithrombin III, prothrombin plasminogen, fibrinogen, factor XIII, immunoglubin G, immunoglubin A, immunoglubin M, immunoglubin D and immunoglubin E, plasmin inhibitor, prothrombin, thrombin, antithrombin, factor V, Factor VII, factor VIII, Factor IX and Factor X containing said lipid soluble process chemicals, said lipid soluble process chemical being a virus attenuating solvent having a high flash point selected from the group consisting of di- or trialkylphosphate having branched or unbranched, substituted or unsubstituted alkyl groups which contain 3 to 30 carbon atoms and mixtures thereof in an amount between 0.01 and 100 mg/ml and a detergent comprising bringing said biological materials containing said lipid soluble process chemicals into contact with an effective amount of a naturally occurring oil extracted from a plant or an animal or a synthetic compound of similar chemical structure, the oil being nonflammable, nonexplosive, compatible with parenterally administered biologics and blood derivatives and pharmaceutically and physiologically tolerable by a human, wherein the oil is contained in an amount of 5 to 50 weight%, based on the weight of the fluid biological materials, agitating the resultant mixture, separating out an upper-phase and a lower-phase by sedimentation or centrifugation and decanting the upper-phase.

The present method involves bringing the biological materials containing the lipid soluble process chemicals, i.e., virus attenuating solvents and detergents into contact with an effective amount of a naturally occurring oil extracted from a plant or an animal or a synthetic compound of similar chemical structure. Thereafter, the resultant mixture is agitated and phase separation occurs by sedimentation or can be facilitated by centrifugation. The upper phase contains oil and extracted process chemicals. The lower phase is relatively free of process chemicals and contains product.

The present invention also concerns whole blood plasma wherein virus is inactivated, blood protein biological activity necessary for use is retained and added virucidal compounds are removed to the extent of 95% or more.

The present invention is thus also directed to a blood plasma having an extent of inactivation of virus greater than 4 logs for said virus, having at least 45% retention of functional activity for particular biologically active blood proteins (e.g. Factor VIII), and having no more than physiologically acceptable levels of lipid soluble process chemicals. The retention of functional activity is relative to untreated blood or blood plasma. The protein composition of the plasma is unchanged (same as if untreated), as measured by cellulose acetate electrophoresis. If, for example, the lipid soluble process chemical is TNBP, the physiologically acceptable levels of TNBP are 1 to 10 ppm. Such physiologically acceptable levels, however, vary depending on the particular lipid soluble process chemical. The lipid soluble process chemicals (added viricidal agents) are removed to the extent of 95% or more. In other words, after treatment of plasma to inactivate virus, the present invention provides for the extraction of harmful solvents used in the virus inactivation in order to produce a safe plasma wherein its therapeutic efficacy is retained.

The present invention also includes protein compositions made from virus sterilized whole plasma.

### BRIEF DESCRIPTION OF THE DRAWINGS

Fig. 1 is a graph showing TNBP in a plasma fraction containing AHF after oil extraction (three extractions using 20%, 10% and 5% ((v/v) oil).
Fig. 2 is a graph showing inactivation of VSV added to AHF by TNBP/"TRITON X 45".

### DETAILED DESCRIPTION OF THE INVENTION

Blood is made up of solids (cells, i.e., erythrocytes, leucocytes, and thrombocytes) and liquid (plasma). The cells contain potentially valuable substances such as hemoglobin, and they can be induced to make other potentially valuable substances such as interferons, growth factors, and other biological response modifiers. The plasma is composed mainly of water, salts, lipids and proteins. The proteins are divided into groups called fibrinogens, serum globulins and serum albumins. Typical antibodies (immune globulins) found in human blood plasma include those directed against infectious hepatitis, influenza H, etc.

Blood transfusions are used to treat anemia resulting from disease or hemorrhage, shock resulting from loss of plasma proteins or loss of circulating volume, diseases where an adequate level of plasma protein is not maintained, for example, hemophilia, and to bestow passive immunization.

Whole blood must be carefully typed and cross matched prior to administration. Plasma, however, does not require prior immunological testing. For certain applications, only a proper fraction of the plasma is required, such as factor VIII for treatment of hemophilia or von Willebrand's disease.

With certain diseases one or several of the components of blood may be lacking. Thus the administration of the proper fraction will suffice, and the other components will not be "wasted" on the patient; the other fractions can be used for another patient. The separation of blood into components and their subsequent fractionation allows the proteins to be concentrated thus permitting concentrates to be treated. Of great importance, too, is the fact that the plasma fractions can be stored for much longer periods than whole blood and they can be distributed in the liquid, the frozen, or the dried state. Finally, it allows salvaging from blood banks the plasma portions of outdated whole blood that are unsafe for administration as whole blood.

Proteins found in human plasma include prealbumin, retinol-binding protein, albumin, alpha-globulins, beta-globulins, gamma-globulins (immune serum globulins), the coagulation proteins (antithrombin III, prothrombin, plasminogen, antihemophilic factor (factor VIII), fibrin-stabilizing factor-factor XIII, fibrinogen), immunoglobins (immunoglobulins G, A, M, D, and E), and the complement components. There are currently more than 100 plasma proteins that have been described. A comprehensive listing can be found in "The Plasma Proteins", ed. Putnam, F.W., Academic Press, New York (1975).

Proteins found in the blood cell fraction include hemoglobin, fibronectin, fibrinogen, enzymes of carbohydrate and protein metabolism, etc. In addition, the synthesis of other proteins can be induced, such as interferons and growth factors.

A comprehensive list of inducible leukocyte proteins can be found in Stanley Cohen, Edgar Pick, J.J. Oppenheim, "Biology of the Lymphokines", Acamedic Press, N.Y. (1979).

Blood plasma fractionation generally involves the use of organic solvents such as ethanol, ether and polyethylene glycol at low temperatures and at controlled pH values to effect precipitation of a particular fraction containing one or more plasma proteins. The resultant supernatant can itself then be precipitated and so on until the desired degree of fractionation is attained. More recently, separations are based on chromotographic processes. An excellent survey of blood fractionation appears in Kirk-Othmer's Encylopedia of Chemical Technology, Third Edition, Interscience Publishers, Volume 4, pages 25 to 62.

The major components of a cold ethanol fractionation are as follows:

| Fraction | Proteins |
|---|---|
| I | fibrinogen; cold insoluble globulin; factor VIII;properdin |
| II and III | IgG; IgM: IgA; fibrinogen;beta-lipoprotein; prothrombin; plasminogen; plasmin inhibitor; factor V; factor VII; factor IX; factor X: thrombin; antithrombin; isoagglutinins; ceruloplasmin; complement C'1, C'3 |
| IV-1 | alpha₁-lipoprotein, ceruloplasmin; plasmin-inhibitor; factor IX; peptidase; alpha-and-beta-globulins |
| IV-4 | transferrin; thyroxine binding globulin; serum esterase; alpha₁-lipoprotein; albumin; alkaline phosphatase |
| V | albumin; alpha-globulin |
| VI | alpha₁-acid glycoprotein; albumin |

The above fractionation scheme can serve as a basis for further fractionations. Fraction II and III, for example, can be further fractionated to obtain immune serum globulin (ISG).

Another fractionation scheme involves use of frozen plasma which is thawed into a cryoprecipitate containing AHF (antihemophilic factor) and fibronectin and a cyrosupernatant. The cryoprecipitate is then fractionated into fibronectin and AHF.

Polyethylene glycol has been used to prepare high purity AHF and non-aggregated ISG.

High risk products with respect to the transmission of hepatitis B and non-A, non-B are fibrinogen, AHF and prothrombin complex, and all other blood protein preparations except immune serum globulin and, because they are pasteurized, albumin solutions.

The methods of the present invention are applicable to biological materials including blood plasma, blood fractions thereof, and blood proteins such as those discussed hereinabove.

Examples of naturally occurring oils extracted from a plant (vegetable, fruit, nut, seed, bean, etc.) or an animal (including fish), i.e., fats and fatty oils, e.g., edible oils, for use in the present invention include soybean and safflower oils, both of which are ingredients of U.S. FDA licensed hyperalimentation fluids for intravenous administration. These preparations are tolerated with no adverse reactions at levels up to 5 g/kg body weight per day during prolonged treatment periods. Thus, vegetable oils that may persist in extracted plasma protein preparations are expected to be innocuous in contrast to residual TNBP in parenteral therapeutics, part of which (up to 4%) may be metabolized to form butanol, which is likely to accumulate and cause neurotoxicity.

Other non-limiting examples of edible vegetable oils for use in the present invention include ricin oil (castor oil), cottonseed oil, corn oil, safflower oil, sesame oil, sunflower seed oil, almond oil, apricot kernel oil, coconut oil, cocoa butter, grapefruit seed oil, linseed oil, mustard seed oil, orange seed oil, palm oil, palm kernel oil, poppyseed oil, rice bran oil, sorghum oil, walnut oil, wheat germ oil, kapok oil, hempseed oil, peanut oil and olive oil. Non-limiting example of animal derived oils for use in the present invention include cow butterfat, goat butterfat, lard, tallow and neatsfoot oil. Furthermore, comparable edible oils derived from fish, e.g., whale oil, cod liver oil, herring oil, sardine oil, menhaden oil and seal oil, or non-toxic mineral oils, etc., may also be used in the present invention for extraction of a variety of virus attenuating organic solvents and/or detergents and/or phorbol esters. Synthetic compounds having similar chemical structures to the aforesaid oils can also be used. A particular preferred synthetic compound is a synthetic triglyceride. Non-limiting examples of synthetic triglycerides for use in the present invention include triolein, tristearin, tripalmitin, trimyristin, and combinations thereof. Particularly preferred is triolein which is pure or a highly defined chemical prepared synthetically from glycerol and oleic acid or from glycerol and defined fatty acid mixtures and is liquid at room temperature. It is also contemplated that synthetic diglycerides and synthetic monoglycerides can be utilized.

The oil for use in the present invention can be any glyceride-containing oil that would be physiologically tolerated.

The lipid soluble process chemicals according to the present invention are ones that are readily extracted from an aqueous solution by using the oils of the present invention.

Although the present invention is applicable to extraction of a wide variety of solvents from said biological materials, the method is particularly suitable for removal of higher boiling solvents.

The use of higher boiling solvents for virus attenuation and removal of these after sufficient contact time by extraction into edible oils avoids the hazards of using flammable solvents altogether whether in the attenuation process or in the process of removing attenuators.

Illustrative members of trialkylphosphates for use in the present invention include tri-(n-butyl) phosphate, tri-(t-butyl) phosphate, tri-(n-hexyl) phosphate, tri-(2-ethylhexyl) phosphate, tri-(n-decyl) phosphate, just to name a few. An especially preferred trialkylphosphate is tri-(n-butyl) phosphate. Mixtures of different trialkylphosphates or mixed alkyl phosphates can also be used. Similarly, the respective dialkylphosphates can be employed, including those of different alkyl group mixtures of dialkylphosphate. Furthermore, mixtures of di- and trialkylphosphates can be employed.

Di- or trialkylphosphates for use in the present invention are employed in an amount preferably between 0.1 mg/ml and 10 mg/ml.

Virus attenuating solvents that can be removed by the method of the present invention may be used for virus inactivation and in the presence of detergents that may not be extractable by methods for solvent removal, as well as those detergents that are extractable.

The di- or trialkylphosphate are used with wetting agents. Such wetting agent can be added either before, simultaneously with, or after the di- or trialkylphosphate contacts the blood biological material, e.g., protein-containing composition. The function of the wetting agent is to enhance the contact of the virus in the biological material, e.g., blood protein-containing composition, with the di- or trialkylphosphate. The wetting agent alone may or may not adequately inactivate the virus.

Contemplated non-ionic detergents for use in the present invention include those which disperse at the prevailing temperature at least 0.1% by weight of the surfactant in an aqueous solution containing the same when introduced therein. In particular there are contemplated detergents which include polyoxyethylene derivatives of fatty acids, partial esters of sorbitol anhydrides, for example, those products known commerically as "TWEEN 80", "TWEEN 10" and "POLYSORBATE 80" and non-ionic oil soluble water detergents such as that sold commercially under the trademark "TRITON X 100", TRITON X 114" and "TRITON X 45" (oxyethylated alkylphenols). Also contemplated are bile salts such as sodium cholate, as well as the "Zwittergents" which are synthetic zwitterionic detergents known as "sulfobetaines", such as N-dodecyl-N, N-dimethyl-2-amino-1 ethane sulphonate and its congeners or non-ionic detergents such as octyl-beta-D-gluco- pyranoside.

Surfactants for use in the present invention may poorly, modestly, or avidly partition into oil phase when oil is admixed with an aqueous solution containing the desire protein biologic together with surfactant.

Preferred surfactants for use in the present invention are those having extraction coefficiencies favoring transfer from aqueous to oil phase with greater than 80% efficiency when the volume of the oil phase is not greater than 20% of the volume of the aqueous phase.

Such extractable detergents include, but are not limited to "TRITON X 45", whereas "TRITON X 110" is not efficiently removable by oil extraction and "TRITON X 114" is marginally removable at the 90% efficiency level, when the volume of extraction oil is 20% of the aqueous volume.

However, less efficient extractions of detergent are contemplated in accordance with the present invention by repeating extractions or by using counter flow extraction techniques until desired levels of detergents have been achieved in the product. Thus, it is contemplated that a variety of detergents may be employed including, but not limited to "TRITON" (octoxanol), nonoxanols, "TWEENS", glucopyranosides, etc.

Treatment of blood protein-containing compositions with trialkylphosphate is effected at a temperature between -5°C and 70°C, preferably between 0°C and 60°C. The time of such treatment (contact) is for at least 1 minute, preferably at least 1 hour and generally 4 to 24 hours. The treatment is normally effective at atmospheric pressure, although subatmospheric and superatmospheric pressures can also be employed.

Exemplary ranges for solvent detergent and oil are as follows:

| | |
|---|---|
| Solvent | 1000-10,000 ppm |
| Detergent | 1000-10,000 ppm |
| Oil | 5-50% of biological fluid (weight %) |

The preferred ranges for each of the above is lowest possible value commensurate with effective virus inactivation and quantitative extraction of solvent and detergent.

Normal and preferred extraction conditions are as follows:

| | |
|---|---|
| normal: | ambient temperature to 35°C, |
| preferred: | ambient, |
| normal exposure time: | 10-60 minutes; |
| preferred: | 30 minutes. |

The present invention is particularly directed, inter alia, to producing a blood plasma protein-containing composition such as, blood plasma, blood plasma fractions, etc., which is substantially free of infectious virus, yet which contains a substantial amount of enzymatically or biologically active (undenatured) protein. More particularly, the present invention is directed to inactivation of lipid-containing virus and preferentially inactivation of AIDS virus (HTLV III/LAV) and hepatitis B, and non-B non-A (NANB) hepatitis virus. Other viruses inactivated during the course of the present invention include, for example, cytomegaloviruses, Epstein Barr viruses, lactic dehydrogenase viruses, herpes groups viruses, rhabdoviruses, leukoviruses, myxoviruses, alphaviruses, arboviruses (group B), paramyxoviruses, arenaviruses, coronaviruses, retroviruses including HTLV III/LAV, and animal leukemia viruses.

Biological fluids for use according to the present invention include, blood plasma, blood plasma fractions, precipitates from blood fractionation and supernatants from blood fractionation, platelet concentrates, white cell (leukocyte) concentrates, and leukocyte-poor packed red cells, as well as platelet rich plasma, platelet concentrates and platelet poor plasma, including packed cell masses comprising the white buffy coat consisting of white blood cells above packed red cells. Also contemplated is the treatment of masses containing concentrates of granulocytes, monocytes, cells capable of producing interferon, tissue necrosis factor and other immune modulators and lymphokines or media separated from such concentrates or suspensions.

Inactivation of virus is obtained by use of the present invention to the extent of at least "4 logs", i.e., virus in a serum is totally inactivated to the extent determined by infectivity studies where that virus is present in the untreated serum in such a concentration that even after dilution to 10⁴, viral activity can be measured.

According to the present invention, there is contemplated a protein-containing composition, particularly whose blood plasma having an extent of inactivation of virus greater than 4 logs of virus, such as AIDS virus, hepatitis B virus and non-A non-B hetaptitis virus, having a retention of functional activity for particularly biologically active proteins of at least 45%, preferably at least 75%, more preferably at least 85%, even more preferably at least 95% and more preferably 98% to 100%, and having no more than physiologically acceptable levels of lipid soluble process chemicals.

The (virus sterilized) whole blood plasma according to the present invention can be transfused directly into a human patient. Alternatively, the (virus sterilized) whole blood plasma according to the present invention can be fractionated to prepare purified plasma protein derivatives (such derivatives can be transfused directly into a human patient).

The whole blood plasma according to the present invention can also be used in cell cultures and as a quality control reagent.

The present invention will now be described with reference to the following non-limiting examples.

### Examples

### Example 1: TNBP (Tri-n-Butylphosphate) Extraction from Aqueous AHF Concentrate

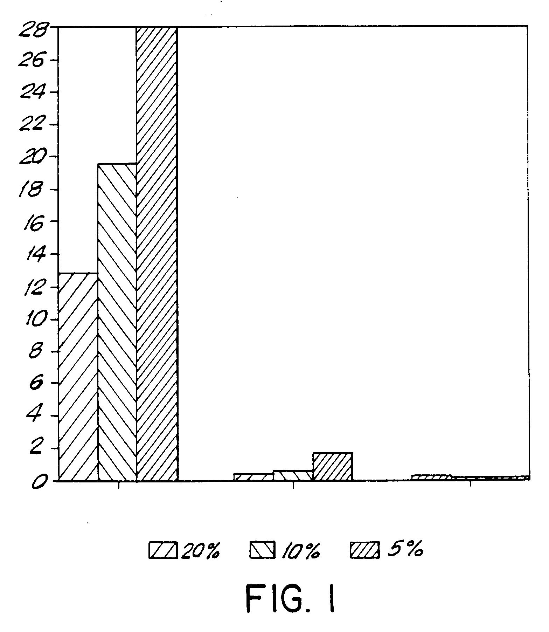

TNBP from Fisher Scientific, Pittsburgh, PA, USA was added to aqueous AHF concentrate in excess and thrice extracted with 5, 10, or 15% (v/v) soybean oil. TNBP remaining in the aqueous phase is represented by Fig. 1.

### Example 2: Extractable and Non-Extractable Detergents

Detergent solutions were twice extracted with equal volumes of soybean oil at ambient temperature. The results of Example 2 as summarized hereinbelow in Table 1.

**TABLE 1**

| Detergent | % Remaining in Aqueous Phase | Extraction |
|---|---|---|
| "TWEEN 80" (from ICI Americas) | 80 | poor extraction |
| "TRITON X 114" (from Rohm & Haas, Philadelphia, PA, USA) | 19 | good extraction |
| "TRITON X 45" (front Rohm & Haas) | < 2 | excellent extraction |
| n-Dodecylglucopyranoside (from Sigma Chemical Co., St. Louis, MO, USA) | 19 | good extraction |

The above Example 2 demonstrates a one-step removal of both solvent and detergent.

### Example 3: Virus Inactivation of AHF Concentrate with TNBP/TRITON X 45 and Reagent Removal by Extraction with Soybean Oil

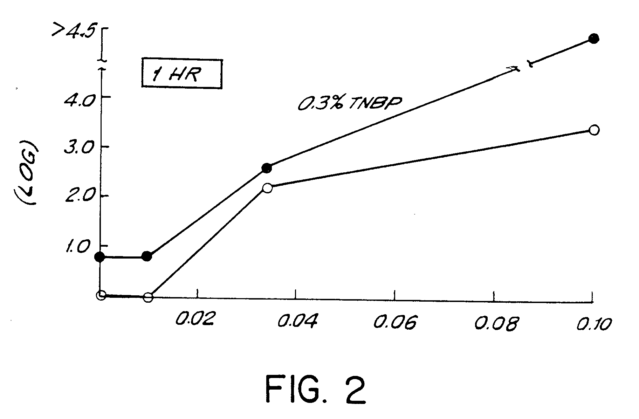

Greater than 10^{4.5} infectious units of vesicular stomatitis virus was added to AHF concentrate solutions containing 0.1% "TRITON X 45" with and without 0.3% TNBP. The detergent alone killed greater than 10^{3.5} units of virus infectivity within 1 hour at 24°C and detergent plus .3% TNBP completely abolished infectivity within the same time. Extraction with 20% soybean oil (v/v) removed greater than 96% of the "TRITON X 45", as well as the TNBP with retention of greater than 85% of the Antihemophilic Factor activity. The results for Example 3 are given in Fig. 2.

### Example 4: Virus Inactivated Single Donor Plasma

Plasma harvested from donated blood in the presence of anticoagulants and transferred to a satellite container, is treated by aseptic addition of solvent or solvent and detergent pairs. The detergent employed should be tolerated at least at the concentrations employed without adverse side reactions when administered intravenously. The solvent is used alone or the solvent/detergent pair as demonstrable by in vitro or in in vivo assays for virus inactivation must be capable of attenuating contaminating virions sufficiently to render them non-infective.

Blood collected into anticoagulant solution was sedimented and about 100 cc of the supernatant plasma was transferred to a flask containing test virus suspensions. A virus attenuating organic solvent was added and finely dispersed by stirring. In this example, the solvent was TNBP 2%. After exposure for four hours at 37°C, the TNBP was extracted into 20 ml cottonseed oil U.S.P. by shaking then separating the phases by sedimentation and decanting the upper-phase. The lower phase plasma was analyzed for virus infectivity and for labile clotting factors.

| Results: | |
|---|---|
| Residual TNBP in plasma: | < 0.3 ppm |

| Virus inactivation: | |
|---|---|
| HTLV-III | non-infective in H9 cells |
| VSV | 10^{4.5} infectious units killed within 1 hour |

| Coagulation Factor Activity Retained: | |
|---|---|
| Prothrombin | > 89% |
| Factor VII activity | > 70% |
| Factor V activity | ≥ 56% |
| Factor IX activity | ≥ 80% |
| Antithrombin III activity | ≥ 95% |

| Cellulose Acetate Electrophoresis: Normal distribution of plasma proteins | |
|---|---|
| Fibrinogen | 5.0 - 8.0% |
| alpha-1-globulins | 2.5 - 3.1% |
| alpha 2 globulins | 8.0 - 10.0% |
| beta globulins | 8.0 - 11.0% |
| gamma globulins | 8.0 - 10.0% |
| albumin | 58 - 65% |

### Example 5: Removal of Virus Inactivating Reagents from AHF Enriched Cryoprecipitate of Plasma

Frozen plasma when thawed at temperatures just slightly above the freezing point forms a gelatinous residue (cryoprecipitate) rich in antihemophilic factor. This material when administered intravenously is capable of correcting congenital deficiencies of two inherited types; hemophilia and Von Willebrand's factor deficiency with attendant risks of viral disease transmission.

Frozen plasma cryoprecipitate harvested from approximately 250 cc plasma units separated from approximately 450 ml voluntary blood donations in anticoagulation solution were individually thawed into normal saline at 50 ml ambient temperature, then continuously admixed with 250 mg "TRITON X 45" and 0.75 ml TNBP for 5 hours. The virus attenuating chemicals were twice extracted into 10 ml cottonseed oil.

### Results: (average of 4 experiments)

The aqueous phase contained less than 3 ppm TNBP and greater then 97% of the "TRITON X 45" had partitioned into the sequential oil phases. AHF procoagulant activity remaining in the aqueous phase ranged from 80-150 international units.

Promotion of platelet aggregation in the presence of ristocetin, a requisite though insufficient indicator of VWF activity, appeared to be normal.

### Example 6: Extraction Of TNBP In The Production Of Virus Inactivated AHF Concentrate

Pooled cryoprecipitate of plasma was extracted with an aqueous solution containing heparin and ionized calcium. The pH was adjusted to 6.4 and the temperature was adjusted to 10°C forming a precipitate which was centrifugally separated and discarded. The supernate after aluminum hydroxide adsorption was exposed to 0.3% w/v TNBP/0.2% sodium cholate for six hours at 30°C with stirring. Soybean oil (0.05 vol : vol) was finely dispersed into the mixture - the phases were separated, the oil layer was removed. The aqueous phase contained less than 20 ppm of the added 3,000 ppm TNBP.

The volume of aqueous phase was reduced by semi-permeable membrane filtration and then passed through a molecular exclusion column of gel ("SEPHADEX G25").

The effluent TNBP level associated with effluent AHF activity was 0.5 ppm/30 IU AHF.

## Claims

1. A method of removing lipid soluble process chemicals from fluid biological materials selected from the group consisting of blood plasma, Fraction I, Fraction II, Fraction III, Fraction IV-1, Fraction IV-4, Fraction V, Fraction VI, fibronectin, antihemophilic factor, prealbumin, retinol-binding protein, albumin, alpha-globulins, beta-globulins, gamma-globulins, antithrombin III, prothrombin, plasminogen, fibrinogen, factor XIII, immunoglubin G, immunoglubin A, immunoglubin M, immunoglubin D and immunoglubin E, plasmin inhibitor, prothrombin, thrombin, antithrombin, factor V, Factor VII, factor VIII, Factor IX and Factor X containing said lipid soluble process chemicals, said lipid soluble process chemical being a virus attenuating solvent having a high flash point selected from the group consisting of di- or trialkylphosphate having branched or unbranched, substituted or unsubstituted alkyl groups which contain 3 to 30 carbon atoms and mixtures thereof in an amount between 0.01 mg/ml and 100 mg/ml, and a detergent comprising bringing said biological materials containing said lipid soluble process chemicals into contact with an effective amount of a naturally occurring oil extracted from a plant or an animal or a synthetic compound of similar chemical structure, the oil being nonflammable, nonexplosive, compatible with parenterally administered biologics and blood derivatives and pharmaceutically and physiologically tolerable by a human, wherein the oil is contained in an amount of 5 to 50 weight%, based on the weight of the fluid biological materials, agitating the resultant mixture, separating out an upper-phase and a lower-phase by sedimentation or centrifugation and decanting the upper-phase.

2. A method according to claim 1, wherein said oil is selected from the group consisting of soybean oil, safflower oil, ricin oil, cottonseed oil, corn oil, peanut oil, olive oil, whale oil and cod liver oil.

3. A method according to claim 1, wherein said solvent is tri-(n-butyl)phosphate.

4. A method according to claim 1, wherein the synthetic compound is a synthetic triglyceride and is selected from the group consisting of tristearin, tripalmitin, triolein, trimyristin and combinations thereof.

## Patentansprüche

1. Verfahren zur Entfernung lipidlöslicher Prozeßchemikalien aus biologischen, diese lipidlöslichen Prozeßchemikalien enthaltenden Materialien, ausgewählt aus der Gruppe, bestehend aus Blutplasma, Fraktion I, Fraktion II, Fraktion III, Fraktion IV-1, Fraktion IV-4, Fraktion V, Fraktion VI, Fibronectin, Antihämophiliefaktor, Präalbumin, Retinol bindendes Protein, Albumin, Alphaglobuline, Betaglobuline, Gammaglobuline, Antithrombin III, Prothrombin, Plasminogen, Fibrinogen, Faktor XIII, Immunoglubin G, Immunoglubin A, Immunoglubin M, Immunoglubin D und Immunoglubin E, Plasmininhibitor, Prothrombin, Thrombin, Antithrombin, Faktor V, Faktor VII, Faktor VIII, Faktor IX und Faktor X, wobei die lipidlösliche Prozeßchemikalie ein Virus schwächendes Lösungsmittel mit einem hohen Flammpunkt, ausgewählt aus der Gruppe, bestehend aus Di- oder Trialkylphosphat mit verzweigten oder unverzweigten, substituierten oder nicht substituierten Alkylgruppen, die 3 bis 30 Kohlenstoffatome enthalten, und Mischungen hieraus in einer Menge zwischen 0,01 mg/ml und 100 mg/ml und ein Detergenz ist, wobei das biologische Material, das die lipidlöslichen Prozeßchemikalien enthält, mit einer wirksamen Menge eines natürlich vorkommenden Öls, das aus einer Pflanze oder einem Tier extrahiert wurde, oder einer synthetischen Verbindung mit einer ähnlichen chemischen Struktur in Berührung gebracht wird, wobei das Öl nicht entflammbar, nicht explosiv, verträglich mit parenteral verabreichbaren biologischen Materialien und Blutderivaten und pharmazeutisch und physiologisch verträglich bei einem Menschen ist, worin das Öl in einer Menge von 5 bis 50 Gew.%, bezogen auf das Gewicht des flüssigen biologischen Materials, enthalten ist, das erhaltene Gemisch geschüttelt wird, eine obere und eine untere Phase durch Sedimentation oder Zentrifugieren getrennt werden und die obere Phase dekantiert wird.

2. Verfahren nach Anspruch 1, worin das Öl ausgewählt ist aus der Gruppe, bestehend aus Sojabohnenöl, Saffloröl, Rizinusöl, Baumwollsamenöl, Maisöl, Erdnußöl, Olivenöl, Walfischöl und Dorschlebertran.

3. Verfahren nach Anspruch 1, worin das Lösungsmittel Tri-(n-butyl)phosphat ist.

4. Verfahren nach Anspruch 1, worin die synthetische Verbindung ein synthetisches Triglycerid ist und ausgewählt aus der Gruppe bestehend aus Tristearin, Tripalmitin, Triolein, Trimyristin und Kombinationen hieraus ist.

## Revendications

1. Procédé d'élimination des produits chimiques de procédé solubles dans les lipides des liquides biologiques choisis dans le groupe constitué du plasma sanguin, de la fraction I, de la fraction II, de la fraction III, de la fraction IV-1, de la fraction IV-4, de la fraction V, de la fraction VI, de la fibronectine, du facteur antihémophilique, de la préalbumine, de la protéine de fixation du rétinol, de l'albumine, des alpha-globulines, des bêta-globulines, des gamma-globulines, de l'antithrombine III, de la prothrombine, du plasminogène, du fibrinogène, du facteur XIII, de l'immunoglobuline G, de l'immunoglobuline A, de l'immunoglobuline M, de l'immunoglobuline D et de l'immunoglobuline E, de l'inhibiteur de plasmine, de la prothrombine, de la thrombine, de l'anti-thrombine, du facteur V, du facteur VII, du facteur VIII, du facteur IX et du facteur X contenant lesdits produits chimiques de procédé solubles dans les lipides, lesdits produits chimiques de procédé solubles dans les lipides étant un solvant d'atténuation des virus ayant un point éclair élevé, choisi dans le groupe constitué des phosphates di- ou trialkyliques, ayant des groupes alkyle ramifiés ou non ramifiés substitués ou non substitués contenant de 3 à 30 atomes de carbone et leurs mélanges en quantité comprise entre 0,01 mg/ml et 100 mg/ml et un détergent, ledit procédé consistant à mettre en contact lesdits liquides biologiques contenant lesdits produits chimiques de procédé solubles dans les lipides avec une quantité efficace d'une huile d'origine naturelle extraite d'une plante ou d'un animal ou avec un composé synthétique de structure chimique semblable, l'huile étant non inflammable, non explosive, compatible avec les liquides biologiques administrés par voie parentérale et les dérivés du sang et tolérables pharmaceutiquement et physiologiquement par un être humain, procédé dans lequel l'huile est présente en quantité allant de 5 à 50% en poids rapportée au poids des liquides biologiques, ledit procédé consistant à agiter ensuite le mélange résultant, à séparer une phase supérieure et une phase inférieure par sédimentation ou centrifugation et à décanter la phase supérieure.

2. Procédé selon la revendication 1, dans lequel ladite huile est choisie dans le groupe constitué de l'huile de soja, de l'huile de carthame, de l'huile de ricin, de l'huile de graines de coton, de l'huile de maïs, de l'huile de cacahuètes, de l'huile d'olive, de l'huile de baleine et de l'huile de foie de morue.

3. Procédé selon la revendication 1, dans lequel ledit solvant est du phosphate tri-(n-butylique).

4. Procédé selon la revendication 1, dans lequel le composé synthétique est une triglycéride synthétique et est choisi dans le groupe constitué de la tristéarine, de la tripalmitine, de la trioléine, de la trimyristine et de leurs combinaisons.
